(19) **Europäisches Patentamt European Patent Office Office européen des brevets**

(11) **EP 4 091 632 A1**

(12) **EUROPEAN PATENT APPLICATION**
published in accordance with Art. 153(4) EPC

(43) Date of publication:
**23.11.2022 Bulletin 2022/47**

(21) Application number: **21741698.1**

(22) Date of filing: **15.01.2021**

(51) International Patent Classification (IPC):
**A61K 45/00** (2006.01)     **A61K 39/395** (2006.01)
**A61P 13/12** (2006.01)     **A61P 25/02** (2006.01)
**A61P 43/00** (2006.01)     **C12N 15/13** (2006.01)

(52) Cooperative Patent Classification (CPC):
**A61K 39/395; A61K 45/00; A61P 13/12;**
**A61P 25/02; A61P 43/00; C07K 16/00**

(86) International application number:
**PCT/JP2021/001261**

(87) International publication number:
**WO 2021/145432 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.01.2020   JP 2020004444**

(71) Applicants:
• **OSAKA UNIVERSITY**
  **Suita-shi**
  **Osaka 565-0871 (JP)**
• **Mitsubishi Tanabe Pharma Corporation**
  **Osaka-shi,**
  **Osaka 541-8505 (JP)**

(72) Inventors:
• **YAMASHITA, Toshihide**
  **Suita-shi, Osaka 565-0871 (JP)**
• **ITOKAZU, Takahide**
  **Suita-shi, Osaka 565-0871 (JP)**
• **UNO, Hiroki**
  **Suita-shi, Osaka 565-0871 (JP)**
• **ISHIDA, Hirokazu**
  **Osaka-shi, Osaka 541-8505 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **AGENT FOR PREVENTION OR TREATMENT OF DIABETIC AUTONOMIC NEUROPATHY**

(57)     This invention provides an agent comprising a RGMa inhibiting substance for preventing or treating diabetic autonomic neuropathy.

EP 4 091 632 A1

**Fig. 3**

## Description

TECHNICAL FIELD

The present invention relates to an agent comprising a RGMa inhibiting substance for preventing or treating diabetic autonomic neuropathy.

BACKGROUND ART

Diabetic neuropathy is one of the three major complications specific to diabetes, which develops early and is frequent. Among them, diabetic autonomic neuropathy is a disorder of autonomic nerve fibers that control organs throughout the body, caused by chronic hyperglycemia, and is a complex disease with a wide variety of signs and symptoms. Diabetic autonomic neuropathy causes abnormalities in cardiovascular, gastrointestinal, urinary and genitourinary, skin, pupil, and adrenal functions, exhibiting orthostatic and dietary hypotension, gastric emptying disorders, bladder and sexual dysfunction, abnormal sweating, abnormal pupils, and unconscious hypoglycemia, respectively (Non-Patent Document 1).

Diabetic nephropathy, which is one of the three major complications of diabetes, progresses to chronic kidney dysfunction as the condition progresses. Diabetic nephropathy is the leading cause of dialysis therapy for end-stage renal failure in Japan and is known to be accompanied by autonomic neuropathy caused by both urinary toxicity and diabetic factors (Non-Patent Document 2, Non-Patent Document 3).

On the other hand, there are cases of poor proteinuria in renal failure patients with diabetes. Such cases may include hypertensive nephrosclerosis complicated by diabetes, but diabetic nephropathy cannot be diagnosed because it is not accompanied by proteinuria. In 2007, the United States defined diabetic kidney disease (DKD) as chronic kidney disease (CKD) for which pathological diagnosis is not a prerequisite and in which diabetes is clinically considered to be involved in the onset or progression of the disease (Non-Patent Document 4) , and the term "diabetic kidney disease" is now used in Japan. Diabetic kidney disease is a concept including diabetic nephropathy (Non-Patent Document 5).

Reflecting such conceptual changes, while past staging assumed that the progression of diabetic nephropathy was accompanied by urinary protein, the presence or absence of urinary protein is currently non-essential for diagnosis of kidney dysfunction complicating diabetes.

RGM (repulsive guidance molecule) is a membrane protein that has been initially identified as an axon guidance molecule in the visual system (see Non Patent Document 6). RGM family includes three members called RGMa, RGMb, and RGMc (Non Patent Document 7). At least RGMa and RGMb are known to work in the same signaling mechanism (Non Patent Document 8). RGMc plays an important role in iron metabolisms.

Subsequent studies have revealed that RGM functions to control, for example, axon guidance and laminar formation in *Xenopus* and chick embryos, and cephalic neural tube closure in mouse embryos (Non Patent Document 9). Patent Document 1 discloses an axon regeneration promoting agent containing an anti-RGM neutralizing antibody as an active ingredient.

In addition to its functions in developmental stages, RGMa is expressed again after central nervous system injuries in an adult human and rat. Further, inhibition of RGMa in rat promotes axon growth after spinal cord injuries and facilitates functional recovery (Non Patent Document 10). From these facts, RGMa is considered as an inhibitor of axon regeneration after central nervous system injuries. Specific examples of antibodies to neutralize RGMa include those described in Patent Document 2 (such as 5F9, and 8D1), Patent Document 3 (such as AE12-1, an AE12-1Y), and Patent Document 4 (such as r116A3, r70E4, r116A3C, and rH116A3).

As described above, roles of RGMa in central nervous system injuries have been reported, however, involvement of RGMa in the treatment of diabetic autonomic neuropathy, especially diabetic nephropathy, has not been identified and no such therapeutic agent has been known.

CITATION LIST

Patent Document

[Patent Document 1] International Publication No. WO2005/087268
[Patent Document 2] International Publication No. WO2009/106356
[Patent Document 3] International Publication No. WO2013/112922
[Patent Document 4] International Publication No. WO2016/175236

Non Patent Document

[Non Patent Document 1] Diabetes Care 26: 1553-1579, 2003
[Non Patent Document 2] Journal of the Japanese Society for Dialysis Therapy 19(9), 905-909, 1986
[Non Patent Document 3] Journal of the Japan Diabetes Society 27(6): 715-721, 1984 [Non Patent Document 4] Am J Kidney Dis 2007: 49: S12-154
[Non Patent Document 5] Evidence-based CKD Medical Guideline 2018, P.104-105 [Non Patent Document 6] Neuron 5, 735-743 (1990).
[Non Patent Document 7] Philos. Trans. R. Soc. Lond. B Biol. Sci., 361: 1513-29, 2006 [Non Patent Document 8] Biochem. Biophys. Res. Commun.,

382, 795-800 (2009).
[Non Patent Document 9] Curr. Opin. Neurobiol., 17, 29-34 (2007).
[Non Patent Document 10] J. Cell Biol., 173, 47-58 (2006).

SUMMARY OF INVENTION

An object of the present invention is to provide an effective drug for diabetic autonomic neuropathy.

The present inventors have intensively studied for achieving the above object and found that a RGMa inhibiting substance, in particular, an anti-RGMa neutralizing antibody has an improving effect on diabetic autonomic neuropathy, thereby completed the present invention.

The present invention is as follows.

1. An agent for preventing or treating diabetic autonomic neuropathy comprising a RGMa inhibiting substance.

2. The preventive or therapeutic agent according to item 1, wherein the diabetic autonomic neuropathy is autonomic neuropathy caused by chronic hyperglycemia or autonomic neuropathy which causes kidney dysfunction.

3. The preventive or therapeutic agent according to item 1 or 2, wherein the diabetic autonomic neuropathy is autonomic neuropathy which causes kidney dysfunction.

4. The preventive or therapeutic agent according to any one of items 1 to 3, wherein the diabetic autonomic neuropathy is a kidney disease which is involved in kidney dysfunction.

5. The agent for preventing or treating according to item 4, wherein the kidney disease which is involved in kidney dysfunction is a chronic kidney disease.

6. The preventive or therapeutic agent according to any one of items 1 to 5, wherein the RGMa inhibiting substance is an anti-RGMa neutralizing antibody.

7. The preventive or therapeutic agent according to item 6, wherein the anti-RGMa neutralizing antibody is a humanized antibody.

8. The preventive or therapeutic agent according to item 6 or 7, wherein the anti-RGMa neutralizing antibody is an antibody recognizing an amino acid sequence selected from SEQ ID NOS: 16, 36, 37, 38 and 39.

9. The preventive or therapeutic agent according to any one of items 6 to 8, wherein the anti-RGMa neutralizing antibody is an antibody selected from the following (a) to (1):

(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9, and an HCDR3 comprising the amino acid sequence represented by SEQ ID NO: 10;

(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15, and an HCDR3 comprising an amino acid sequence comprising SFG;

(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;

(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27, and an HCDR3 comprising the amino acid sequence represented by SEQ ID NO: 28;

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and

(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34.

10. A method of preventing or treating diabetic autonomic neuropathy, which comprises administration of an effective dose of a RGMa inhibiting substance to a mammal in need of treatment.

11. The preventive or therapeutic method according to item 10, wherein the RGMa inhibiting substance is an anti-RGMa neutralizing antibody.

12. Use of a RGMa inhibiting substance in manufacture of an agent for preventing or treating diabetic autonomic neuropathy.

13. The use according to item 12, wherein the RGMa inhibiting substance is an anti-RGMa neutralizing antibody.

Effects of Invention

According to the present invention, a RGMa inhibiting substance, in particular. an anti-RGMa neutralizing antibody, is useful as an agent for preventing or treating diabetic autonomic neuropathy.

BRIEF DESCRIPTION OF THE DRAWINGS

Fig. 1 is a diagram illustrating changes in RGMa gene expression in a kidney under diabetic pathology.
Fig. 2 is representative staining images (drawing substitute photos). Upper left: non-diabetic, control antibody group; upper right: non-diabetic, anti-RGMa neutralizing antibody group; lower left: diabetic, control antibody group; lower right: diabetic, anti-RGMa neutralizing antibody group.
Fig. 3 is a diagram illustrating quantitative data of TH fiber density.
Fig. 4 is a diagram illustrating quantitative data for the urinary albumin/urinary creatinine ratio.

DETAILED DESCRIPTION OF THE INVENTION

The terms used in the present invention will be described below.

[Neutralization]

The term "neutralization" as used herein refers to an action of binding to an objective target and inhibiting functions of the target. For example, a RGMa inhibiting substance refers to a substance which binds to RGMa and consequently acts to inhibit a biological activity of RGMa.

[Epitope]

As used herein, the term "epitope" includes a polypeptide determinant that can specifically bind to an immunoglobulin or a T-cell receptor. In some embodiments, an epitope can include a chemically active group on the surface of the molecule (for example, an amino acid, a sugar side chain, phosphoryl or sulfonyl). In some embodiments, an epitope can have particular characteristics of three-dimensional structure and/or electric charge. Epitopes refer to regions in antigens, to which antibodies bind.

[Isolated]

As used herein, the term "isolated" such as in isolated RGMa inhibiting substance (for example, antibody) means being identified, and separated and/or recovered from components in natural states. Impurities in natural states are substances that can interfere with the diagnostic or therapeutic use of the antibody, including enzymes, hormones and other proteinous or nonproteinous solutes. Generally, RGMa inhibiting substances or the like may be isolated through at least one purification step. RGMa inhibiting substances purified through at least one purification step can be referred to as "isolated RGMa inhibiting substances".

[Antibody]

As used herein, the term "antibody" refers broadly to an immunoglobulin (Ig) molecule comprising four polypeptide chains, namely two heavy chains (H chains) and two light chains (L chains), that substantially retain the characteristics of an Ig molecule to bind to an epitope.

[Human Antibody]

As used herein, the term "human antibody" refers to an antibody comprising light and heavy chains which are both derived from human immunoglobulins. Depending on the difference in the constant region of the heavy chain, human antibodies include IgG comprising a γ heavy chain (including IgG1, IgG2, IgG3 and IgG4); IgM comprising a μ heavy chain; IgA comprising an α heavy chain (including IgA1 and IgA2); IgD comprising a δ heavy chain; and IgE comprising an ε heavy chain. In principle, a light chain comprises either κ or λ chain.

[Humanized Antibody]

As used herein, the term "humanized antibody" refers to an antibody comprising variable regions comprising complementarity determining regions from antibodies derived from non-human animals and framework regions derived from human antibodies, and constant regions derived from human antibodies.

[Chimeric Antibody]

As used herein, the term "chimeric antibody" refers to an antibody in which the light chain, the heavy chain, or both comprise a non-human derived variable region and a human derived constant region.

[Monospecific Antibody]

As used herein, the term "monospecific antibody" refers to an antibody comprising a single independent antigen recognition site having a single antigen specificity. For example, a monospecific antibody that recognizes RGMa may be referred herein to as a RGMa-monospecific antibody.

[Multispecific Antibody]

As used herein, the term "multispecific antibody" refers to antibodies comprising two or more independent antigen recognition sites having two or more different antigen specificities, including bispecific antibodies having two antigen specificities and trispecific antibodies having three antigen specificities.

[Complementarity Determining Region (CDR)]

The term "complementarity determining region (CDR)" refers to a region forming an antigen binding site in a variable region of an immunoglobulin molecule, which is also called a hypervariable region, and particularly refers to a portion in which the amino acid sequence changes greatly for each immunoglobulin molecule. As CDRs, light and heavy chains each have three CDRs. Three CDRs contained in a light chain may be referred to as LCDR1, LCDR2, and LCDR3, while three CDRs contained in a heavy chain may be referred to as HCDR1, HCDR2, and HCDR3. For example, CDRs of an immunoglobulin molecule are assigned according to the Kabat numbering system (Kabat, et al., 1987, Sequences of Proteins of Immunological Interest, US Department of Health and Human Services, NIH, USA).

[Effective Dose]

The term "effective dose" refers to a sufficient dose of a preventive or therapeutic agent to alleviate or ameliorate the severity and/or duration of a disorder, or one or more symptoms thereof; to prevent progression of the disorder; to reverse the disorder; to prevent the recurrence, onset, development, or progression of one or more symptoms associated with the disorder; to detect the disorder; or to enhance or improve one or more preventive or therapeutic effects of another therapy (for example, a preventive drug or a therapeutic drug).

[Percent (%) Identity of Amino Acid Sequence]

"Percent (%) identity" of the amino acid sequence of a candidate polypeptide sequence, such as a variable region, with respect to the amino acid sequence of a reference polypeptide sequence is defined as a percent of the same amino acid residues in the candidate sequence as the amino acid residues in the particular reference polypeptide sequence, which percent is obtained by arranging the sequences and introducing gaps if necessary to obtain maximal % identity, without considering any conservative substitutions as part of the sequence identity. Alignment for determination of % identity can be accomplished by using various methods within the skill of the art, for example, using a publicly available computer software, such as BLAST, BLAST-2, ALIGN, or Megalign (DNASTAR) software. Those skilled in the art can determine appropriate parameters for sequence alignment, including algorithm needed to achieve maximal alignment over the full length of the sequence to be compared. However, for the purposes herein, values of % identity are obtained in pairwise alignment using a computer program for comparing sequences, BLAST.

When BLAST is used in comparison of amino acid sequences, the % identity of a given amino acid sequence A to a given amino acid sequence B is calculated as follows:

$$a \text{ fraction } X/Y \text{ multiplied by } 100$$

where X is the number of amino acid residues scored as identical in a programmed alignment of A and B by the sequence alignment program Blast, and Y is the total number of amino acid residues in B. It will be understood that when the lengths of amino acid sequences A and B are different, the % identities of A to B and of B to A are different. Unless stated otherwise, all % identity values herein are obtained using a computer program BLAST, as described in the immediately preceding paragraph.

[Conservative Substitution]

"Conservative substitution" means replacement of an amino acid residue with another chemically similar amino acid residue so as not to substantially alter the activity of the peptide. Examples thereof include a case where a hydrophobic residue is substituted with another hydrophobic residue, and a case where a polar residue is substituted with another polar residue having the same charge. Examples of functionally similar amino acids eligible for such substitution include, as for non-polar (hydrophobic) amino acids, alanine, valine, isoleucine, leucine, proline, tryptophan, phenylalanine, and methionine. As for polar (neutral) amino acids, they include glycine, serine, threonine, tyrosine, glutamine, asparagine, and cysteine. As for positively charged (basic) amino acids, they include arginine, histidine, and lysine. As for negatively charged (acidic) amino acids, they include aspartic acid, and glutamic acid.

The present invention will be described in detail below.

The present invention provides an agent for preventing or treating diabetic autonomic neuropathy, which is a novel use of a RGMa inhibiting substance.

Further, the present invention provides a method of preventing or treating diabetic autonomic neuropathy, comprising a step of administering a preventive or therapeutic agent comprising an effective dose of a RGMa inhibiting substance to a mammal in need of treatment.

<RGMa Inhibiting substance>

There is no particular restriction on the RGMa inhibiting substance of the present invention, insofar as it is a substance that acts on RGMa itself to inhibit or reduce the activity of RGMa (hereinafter sometimes referred to simply as "RGMa activity"). For example, a substance having an activity that directly inhibits (or reduces) the RGMa activity by binding to the RGMa, or an activity that indirectly inhibits (or reduces) the RGMa activity by inhibiting the binding of a RGMa to a receptor (e.g., the compound, antibody, etc. described below) is referred to as the RGMa inhibiting substance of the present invention.

The RGMa inhibiting substance of this invention may also be a substance that inhibits the expression of RGMa, for example, a substance that inhibits the expression of RGMa to inhibit (reduces) the RGMa activity (e.g., a nucleic acid molecule described below) is also included in the RGMa inhibiting substance of the present invention.

RGMa is identified as a protein that inhibits neurite outgrowth in the central nervous system. A human RGMa protein is biosynthesized as a precursor protein comprising 450 amino acids as shown in SEQ ID NO: 1. The signal peptide from Met 1 to Pro 47 present at the N terminus (which refers to the peptide from the methionine residue at position 1 to the proline residue at position 47 counted from the N-terminal side, and is hereafter described as above) is removed. Then, the peptide bond between Asp 168 and Pro 169 is cleaved to generate an N-terminal domain. In the C-terminal fragment from Pro 169, the peptide from Ala 425 to Cys 450 at the C terminus is removed so that Ala 424 becomes the C terminus. Then, a GPI anchor is added to the C-terminal carboxyl group of Ala 424 to generate a C-terminal domain. The human RGMa protein is expressed on the cell membrane via a GPI anchor as a mature protein in which the N-terminal domain (Cys 48 to Asp 168) and the C-terminal domain (Pro 169 to Ala 424) are linked by a disulfide bond.

RGMa in the present invention may be derived from any animals. Preferably, RGMa is human RGMa. A human RGMa precursor protein comprises the amino acid sequence shown in SEQ ID NO: 1 in Sequence Listing. A mouse RGMa precursor protein comprises the amino acid sequence shown in SEQ ID NO: 2 in Sequence Listing, while a rat RGMa precursor protein comprises the amino acid sequence shown in SEQ ID NO: 3 in Sequence Listing. Removal of the C-terminal peptides from each amino acid sequence results in mature proteins having the same amino acid sequence, respectively.

RGMa genes include, but are not limited to, a human RGMa gene comprising the nucleotide sequence shown in SEQ ID NO: 4. Nucleotide sequences of RGM genes derived from various organisms can be easily obtained from known databases (for example, GenBank).

Specific examples of the RGMa inhibiting substance of the present invention include low molecular weight compounds, anti-RGMa neutralizing antibodies, and functionally modified antibodies thereof, conjugated antibodies thereof, and antigen-binding fragments thereof, as well as an siRNA (short interfering RNA), an shRNA (short hairpin RNA), and an antisense oligonucleotide, which are nucleic acid molecules of RGMa. Among these RGMa inhibiting substances, an anti-RGMa neutralizing antibody, a functionally modified antibody thereof, a conjugated antibody thereof, and an antigen-binding fragment thereof are preferable, an anti-RGMa neutralizing antibody, and an antigen-binding fragment thereof are more preferable, and an anti-RGMa neutralizing antibody is especially preferable.

<Anti-RGMa Neutralizing Antibody>

According to the present invention, the anti-RGMa neutralizing antibody is an antibody that binds to RGMa to neutralize the RGMa activity, and may be a polyclonal, or monoclonal antibody. A monoclonal antibody is more preferable according to the present invention. The anti-RGMa-neutralizing antibody of the present invention may be a RGMa-monospecific antibody, or a multispecific antibody that recognizes RGMa and a plurality of other antigens. A RGMa-monospecific antibody is more preferable.

Specifically, the epitopes in human RGMa are preferably one or more of SEQ ID NO: 16 (amino acid numbers 47 to 69 in SEQ ID NO: 1), SEQ ID NO: 36 (amino acid numbers 298 to 311 in SEQ ID NO: 1), SEQ ID NO: 37 (amino acid numbers 322 to 335 in SEQ ID NO: 1), SEQ ID NO: 38 (amino acid numbers 349 to 359 in SEQ ID NO: 1), and SEQ ID NO: 39 (amino acid numbers 367 to 377 in SEQ ID NO: 1); more preferably a combination of SEQ ID NOS: 36 and 37;, and particularly preferably a combination of SEQ ID NOS: 36, 37 and 39.

The anti-RGMa neutralizing antibodies of the present invention include polyclonal or monoclonal antibodies obtained by immunizing mammals such as mice with an antigen which is a RGMa protein or a partial fragment thereof (for example, epitope fragment described above); chimeric antibodies and humanized antibodies produced by gene recombination technology; and human antibodies produced, for example, by a transgenic animal producing human antibody. When the antibody of the present invention is administered to a human as a medicine, the antibody is desirably a humanized antibody or a human antibody from the viewpoint of reducing side effects.

Specific examples of the anti-RGMa neutralizing antibody of the present invention include the following antibodies (a) to (1). As the respective production methods therefor, the methods described in Patent Documents 2 to 4 may be used.

Examples thereof include antibodies to be selected from:

(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6 and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9 and an HCDR3 comprising the amino acid sequence represented by SEQ ID NO: 10 (the anti-RGMa neutralizing antibody also includes antibodies whose epitopes are the amino acid sequences represented by SEQ ID NOS: 36, 37 and 39);

(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12 and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15 and an HCDR3 comprising an amino acid sequence comprising SFG (the anti-RGMa neutralizing antibody also includes antibodies whose epitopes are the amino acid sequences represented by SEQ ID NOS: 36, 37 and 38);

(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18 and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21 and an HCDR3 comprising the amino acid sequence represented by SEQ ID NO: 22;

(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24 and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27 and an HCDR3 comprising the amino acid sequence represented by SEQ ID NO: 28;

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16); and

(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16).

Among these the antibody according to (a) is particularly preferable.

The anti-RGMa neutralizing antibodies of the present invention can be produced using production methods that are conventionally and commonly used. Antigens may be directly used for immunization, or may be used as a complex with a carrier protein. For preparing a complex of an antigen and a carrier protein, condensing agents such as glutaraldehyde, carbodiimides, and maleimide active esters can be used. Examples of the carrier protein include bovine serum albumin, thyroglobulin, hemocyanin, and KLH.

Examples of the mammal to be immunized include mice, rats, hamsters, guinea pigs, rabbits, cats, dogs, pigs, goats, horses and cattle. Examples of the inoculation method include subcutaneous, intramuscular and intraperitoneal administrations. When administered, antigens may be administered in mixture with complete or incomplete Freund's adjuvant, and are usually administered once every 2 to 5 weeks. Antibody-producing cells obtained from the spleen or lymph nodes of the immunized animals are fused with myeloma cells and isolated as hybridomas. As the myeloma cells, those derived from mammals such as mouse, rat, and human are used.

<Polyclonal Antibody>

The polyclonal antibodies can be obtained, for example, from a serum obtained from an immunized animal which is the mammal as described above immunized with the antigen as described above, if necessary in combination with a Freund's adjuvant.

<Monoclonal Antibody>

Specifically, the monoclonal antibodies can be obtained, for example, as follows. That is, the antigen as described above is used as an immunogen, and the immunogen is injected or transplanted once or several times in combination with a Freund's adjuvant, as necessary, to the mammal as described above subcutaneously, intramuscularly, intravenously, into a footpad, or intraperitoneally for immunization. Typically, the immunization is performed about once to four times every 1 day to 14 days from the initial immunization, and after about 1 day to 5 days from the final immunization, antibody-producing cells are obtained from the immunized mammal.

The monoclonal antibodies can be obtained by a method well known to a person skilled in the art (for example, "Current Protocols in Molecular Biology" (John Wiley & Sons (1987)), or Antibodies: A Laboratory Manual, Ed. Harlow and David Lane, Cold Spring Harbor Laboratory (1988)).

The "hybridomas" secreting monoclonal antibodies can be prepared according to the method of Köhler and Milstein, et al. (Nature, 256, 495, 1975) or a modified method based on it. That is, they are prepared by cell fusion between an antibody-producing cell included in the spleen etc. obtained from an immunized mammal, and a myeloma cell, which is not capable of producing an autoantibody, and derived from a mammal, preferably mouse, rat or human.

Examples of the myeloma cell which can be used in the cell fusion include mouse-derived myelomas such as P3/X63-AG8.653 (653), P3/NSI/1-Ag4-1 (NS-1), P3/X63-Ag8.U1 (P3U1), SP2/0-Ag14 (Sp2/O, Sp2), PAI, F0 and BW5147; rat-derived myelomas such as 210RCY3-Ag.2.3.; and human-derived myelomas such as U-266AR1, GM1500-6TG-A1-2, UC729-6, CEM-AGR, D1R11 and CEM-T15.

Examples of the fusion promoter include polyethylene glycols. The cell fusion can usually be performed by a reaction in a temperature range from 20°C to 40°C, preferably from 30°C to 37°C, for about 1 minute to 10 minutes usually using a polyethylene glycol (with an average molecular weight from 1000 to 4000) with a concentration from about 20% to 50%, wherein the ratio of the number of antibody-producing cells to the number of myeloma cells is usually from about 1:1 to 10:1

Screening of hybridoma clones producing the monoclonal antibodies can be carried out by culturing the hybridomas, for example, in a microtiter plate and assaying the culture supernatants in the wells for the reactivity against an immunogen by an immunochemical method such as ELISA.

In the screening of antibody-producing hybridomas, whether the antibody inhibits the RGMa activity in the present invention is also determined in addition to the binding assay with a RGMa protein. The screening methods allow for selection of the anti-RGMa neutralizing antibody of the present invention.

Clones can be further obtained from the wells containing hybridomas producing the desired antibodies by cloning with limiting dilution. Hybridomas are usually selected and grown in an animal cell culture medium containing 10% to 20% fetal bovine serum, supplemented with HAT (hypoxanthine, aminopterin and thymidine).

The monoclonal antibodies can be produced from the hybridomas by culturing the hybridomas *in vitro,* or growing them *in vivo,* for example, in ascitic fluids of mammals such as mice and rats, and isolating the monoclonal antibodies from the resulting culture supernatants or the ascitic fluids of the mammals.

When cultured *in vitro,* it is possible to use a nutrient medium suitable for growing, maintaining, and conserving hybridomas corresponding to various conditions such as the characteristics of the cell species to be cultured, or the culturing method, and producing a monoclonal antibody in the culture supernatant. Examples of the nutrient medium may include a known nutrient medium, or a nutrient medium prepared from a basal medium.

Examples of the basal medium include low-calcium media such as Ham's F12 medium, MCDB 153 medium, and low-calcium MEM medium, and high-calcium media such as MCDB 104 medium, MEM medium, D-MEM medium, RPMI 1640 medium, ASF 104 medium, and RD medium. The basal medium can contain, for example, sera, hormones, cytokines and/or various inorganic or organic substances according to the purpose.

The monoclonal antibodies can be isolated and purified by, for example, subjecting the above-described culture supernatant or ascitic fluid to saturated ammonium sulfate, euglobulin precipitation, a caproic acid treatment, a caprylic acid treatment, an ion exchange chromatography (such as DEAE or DE52), or an affinity column chromatography, for example with an anti-immunoglobulin column or a protein A column. Specifically, the monoclonal antibodies may be purified by any method known as an immunoglobulin purification method, and the purification can be easily achieved by means such as ammonium sulfate fractionation, PEG fractionation, ethanol fractionation, a method utilizing an anion exchanger, and further an affinity chromatography using a RGMa protein.

The monoclonal antibodies can also be obtained by a phage display method. In a phage display method, phages selected from an optional phage antibody library are screened using a desired immunogen, and phages having a desired binding ability to the immunogen are selected. Next, the antibody-corresponding sequence contained in the phage is isolated or sequenced. Based on the information of the isolated sequence or the determined sequence by the sequencing, an expression vector comprising a nucleic acid molecule encoding the antibody or antigen binding domain is constructed. The expression vector is then transfected into a cell line, and the cell line can be cultured to produce a monoclonal antibody. When a human antibody library is used as the phage antibody library, a human antibody having a desired binding ability can be produced.

<Nucleic Acid Molecule>

A nucleic acid molecule encoding an anti-RGMa neutralizing antibody of the present invention or an antigen-binding fragment thereof can be obtained, for example, by the following method. First, total RNA is prepared from a cell such as hybridoma using a commercially available RNA extraction kit, and subsequently cDNAs are synthesized with a reverse transcriptase using random primers and the like. Next, by a PCR method using, as primers, oligonucleotides of sequences respectively conserved in the variable regions in the known human antibody heavy chain and light chain genes, cDNAs encoding the antibody are amplified. Sequences encoding the constant regions can be obtained by amplification of known sequences by a PCR method. The nucleotide sequence of the DNA can be sequenced by a conventional method, for example, by incorporating it into a plasmid for sequencing.

Alternatively, a DNA encoding the monoclonal antibody of the present invention can also be obtained by chemically synthesizing sequences of the variable regions or parts thereof and joining them to sequences comprising the constant regions.

The nucleic acid molecule may encode all of the constant regions and the variable regions of heavy and light chains, or may encode only the variable regions of heavy and light chains. In the case of encoding all of the constant regions and the variable regions, the nucleotide sequences of the constant regions of heavy and light chains are preferably those described in Nucleic Acids Research, vol. 14, p1779, 1986; The Journal of Biological Chemistry, vol. 257, p1516, 1982; or Cell, vol. 22, p197, 1980.

<Functionally Modified Antibody>

A functionally modified antibody of an anti-RGMa neutralizing antibody is prepared by the following method. For example, when an anti-RGMa neutralizing antibody under this application is produced using CHO cells as host cells, in which the $\alpha$1,6-fucosyl transferase (FUT8) gene is destructed, the fucose content in the sugar chain is decreased, and an antibody with an increased cell killing function is obtained. Meanwhile, when the same is produced using CHO cells as host cells, in which the FUT8 gene is transduced an antibody with a weak cell killing function is obtained (International Publication Nos. WO2005/035586, WO2002/31140, and WO00/61739). Meanwhile, the complement activation function can be modulated by modifying the amino acid residues in the Fc region (U.S. Patent Nos. 6,737,056, 7,297,775, and 7,317,091). Further, when the variant of the Fc region with enhanced binding to FcRn, which is one of Fc receptors, is used, the half-life in blood can be prolonged (Hashiguchi Shuhei, et al, Seikagaku, 2010, Vol. 82 (8), p710). These functionally modified antibodies can be produced by genetic engineering.

<Conjugated Antibody>

Examples of a modified molecule of the anti-RGMa neutralizing antibody of the present invention include a conjugated antibody. Examples of the conjugated antibody include a conjugated antibody in which aan anti-RGMa neutralizing antibody is bound chemically or by a genetic engineering technique to a functional molecule other than the present anti-RGMa neutralizing antibody such as nonpeptidic polymers such as polyethylene glycol (PEG), radioactive substances, toxins, low molecular weight compounds, cytokines, growth factors (such as TGF-$\beta$, NGF, and neurotrophin), albumins, enzymes, and other antibodies.

When PEG is bound as the functional molecule, PEG having, but without limitation, a molecular weight from 2,000 Da to 100,000 Da, more preferably from 10,000 Da to 50,000 Da can be used. PEG may be linear or branched. PEG can be bound to the N-terminal amino group in the amino acids of an anti-RGMa neutralizing antibody, etc., for example by using an NHS active group.

When a radioactive substance is used as the functional molecule, its examples include $^{131}$I, $^{125}$I, $^{90}$Y, $^{64}$Cu, $^{99}$Tc, [77]Lu, and [211]At. The radioactive substance can be directly bound to the anti-RGMa neutralizing antibody by a chloramine-T method or the like.

When a toxin is used as the functional molecule, examples thereof include bacterial toxins (such as diphtheria toxin), phytotoxins (such as ricin), low molecular weight toxins (such as geldanamycin), maytansinoids and calicheamicins.

When a low molecular weight compound is used as the functional molecule, examples include daunomycin, doxorubicin, methotrexate, mitomycin, neocarzinostatin, vindesine, and fluorescent dyes such as FITC.

When an enzyme is used as the functional molecule, examples include luciferases (such as firefly luciferases, and bacterial luciferases; US Patent No. 4,737,456), malate dehydrogenases, ureases, peroxidases (such as horseradish peroxidase (HRPO)), alkaline phosphatases, $\beta$-galactosidases, glucoamylases, lysozymes, saccharide oxidases (such as glucose oxidase, galactose oxidase, and glucose-6-phosphate dehydrogenase), heterocyclic oxidases (such as uricase, and xanthine oxidase), lactoperoxidases, and microperoxidases.

Examples of linkers used for chemically bonding the toxin, low molecular weight compound, or enzyme include divalent radicals (such as alkylene, arylene, and heteroarylene), linkers represented by $-(CR_2)_nO(CR_2)_n-$ (wherein R is any substituent, and n is a positive integer), alkoxy repeating units (such as polyethyleneoxy, PEG, and polymethyleneoxy), alkylamino (such as polyethyleneamino, and Jeffamine(trademark)), and diacid esters and amides (including succinate, succinamide, diglycolate, malonate, and caproamide). Chemical modification methods for binding functional molecules have already been established in this field (D. J. King., Applications and Engineering of Monoclonal Antibodies., 1998, T.J. International Ltd, Monoclonal Antibody-Based Therapy of Cancer., 1998, Marcel Dekker Inc.; Chari, et al., Cancer Res., 1992 Vol. 152: 127; and Liu, et al., Proc Natl Acad Sci USA., 1996 Vol 93: 8681).

<Antigen-binding Fragment>

In embodiments of the present invention, "antigen-binding fragments" of antibodies means partial regions having antigen binding properties derived from the antibodies as described above. Specific examples of the fragments include F(ab')$_2$, Fab', Fab, Fv (variable fragment of antibody), disulfide-linked Fv, single-chain antibodies (scFv), and polymers thereof. Examples of the antigen-binding fragments also include conjugated fragments that bind, chemically or by genetic engineering, functional molecules other than the present anti-RGMa neutralizing antibody, such as nonpeptidic polymers, e.g. polyethylene glycol (PEG), radioactive substances, toxins, low molecular weight compounds, cytokines, growth factors (*e.g.* TGF-β, NGF, and neurotrophin), albumins, enzymes, and other antibodies.

The terms "F(ab)$_2$" and "Fab" mean antibody fragments produced by treating an immunoglobulin with pepsin or papain which are proteases, namely produced by digestion at the upstream or downstream side of the disulfide bond existing between two heavy chains in the hinge region. For example, when IgG is treated with papain, it is cleaved at the upstream side of the disulfide bond existing between the two heavy chains in the hinge region to produce two homologous antibody fragments each comprising a light chain comprising a VL (light chain variable region) and a CL (light chain constant region) and a heavy chain fragment comprising a VH (heavy chain variable region) and a CHγ1 (γ1 region in the heavy chain constant region), in which the light chain and the heavy chain fragment are linked to each other via a disulfide bond at the C-terminal domain. Each of the two homologous antibody fragments is referred to as Fab. Meanwhile, when IgG is treated with pepsin, it is cleaved at the downstream side of the disulfide bond existing between the two heavy chains in the hinge region to produce an antibody fragment that is slightly larger than the two Fab linked to each other at the hinge region. This antibody fragment is referred to as F(ab')$_2$.

<Chimeric Antibody>

In a preferred mode of the anti-RGMa neutralizing antibody of the present invention, there is a chimeric antibody. Examples of the "chimeric antibody" include those in which the variable regions are derived from immunoglobulins from non-human animals (such as mouse, rat, hamster and chicken) and the constant regions are derived from human immunoglobulins. The chimeric antibody can be prepared, for example, by immunizing a mouse with the antigen, cleaving out a variable region that binds to the antigen from the gene encoding the mouse monoclonal antibody, and combining the variable region with a constant region of an antibody derived from human bone marrow. The constant region derived from a human immunoglobulin has a unique amino acid sequence depending on each isotype, such as IgG (IgG1, IgG2, IgG3, or IgG4), IgM, IgA (IgA1, or IgA2), IgD, or IgE. The constant region of the recombinant chimeric antibody according to the present invention may be a constant region of a human immunoglobulin belonging to any of the isotypes. The constant region is preferably a constant region of human IgG The chimeric antibody gene thus prepared can be used to prepare an expression vector. A host cell is transformed with the expression vector to obtain a transformed cell that produces the chimeric antibody. Subsequently, the transformed cell is cultured to obtain the desired chimeric antibody from the culture supernatant.

<Humanized Antibody>

In another preferred mode of the anti-RGMa neutralizing antibody of the present invention, there is a humanized antibody. The "humanized antibody" according to the present invention is an antibody obtained by grafting only the DNA sequence of the antigen binding sites (CDRs; complementarity determining regions) of an antibody derived from a nonhuman animal such as mouse to a human antibody gene (CDR grafting). The humanized antibody can be prepared according to the methods described in, for example, Japanese Translated PCT Patent Application Laid-open No. 1992-506458 and Japanese Patent No. 2912618. Specifically, the humanized antibody comprises CDRs partly or wholly derived from monoclonal antibodies from non-human mammals (such as mouse, rat, and hamster); framework regions of variable regions derived from human immunoglobulins; and constant regions derived from human immunoglobulins.

The humanized antibody according to the present invention can be produced, for example, as described below. Needless to say, however, the production method is not limited thereto.

For example, a recombinant humanized antibody derived from a mouse monoclonal antibody can be produced by genetic engineering with reference to Japanese Translated PCT Patent Application Laid-open No. 1992-506458 and Japanese Laid-open Patent Application (Kokai) No. 1987-296890. Specifically, DNA encoding the region of CDRs of a mouse heavy chain and DNA encoding the region of CDRs of a mouse light chain are isolated from hybridomas producing a mouse monoclonal antibody. Further, a human heavy chain gene encoding the whole region other than CDRs of the human heavy chain and a human light chain gene encoding the whole region other than CDRs of the human light chain are isolated from a human immunoglobulin gene.

The isolated DNA encoding the region of CDRs of the mouse heavy chain is grafted to the human heavy chain gene, and the product is introduced into an appropriate expression vector so that it is expressible. Similarly, the DNA encoding the region of CDRs of the mouse light chain is grafted to the human light chain gene, and the product is introduced into another appropriate expression vector so that it is expressible. Alternatively, the human heavy and light chain genes to which mouse CDRs are grafted may be introduced into the same expression vector so that they are expressible. A host cell is transformed with the expression vector thus prepared to obtain a transformed cell producing the humanized antibody. Subsequently, the transformed cell is cultured to obtain the desired humanized antibody from the culture supernatant.

<Human Antibody>

In another preferred mode of the anti-RGMa neutralizing antibody of the present invention, there is a human antibody. The term "human antibody" refers to an antibody in which the entire region including heavy chain variable regions and heavy chain constant regions and light chain variable regions and light chain constant regions constituting the immunoglobulin are derived from genes encoding human immunoglobulins. Human antibodies can be prepared by introducing human antibody genes into mice. Human antibodies can be produced in the same manner as the above-described method for preparing polyclonal antibodies or monoclonal antibodies. Specifically, for example, the method comprises introducing at least human immunoglobulin genes into gene loci of a non-human mammal such as mouse to prepare a transgenic animal and immunizing the transgenic animal with an antigen.

For example, a transgenic mouse that produces a human antibody can be prepared according to the methods described, for example, in Nature Genetics, Vol. 7, p.13-21, 1994; Nature Genetics, Vol. 15, p.146-156, 1997; Japanese Translated PCT Patent Application Laid-open Nos. 1992-504365 and 1995-509137; WO 94/25585; Nature, Vol. 368, p.856-859, 1994; and Japanese Translated PCT Patent Application Laid-open No. 1994-500233. More specifically, for example, HuMab (registered trademark) Mouse (Medarex, Princeton, NJ), KMTM mouse (Kirin Pharma Company, Japan), and KM (FCγRIIb-KO) mouse may be used.

Specific examples of the anti-RGMa neutralizing antibody of the present invention include those having CDRs comprising specific amino acid sequences in the heavy chain variable region, and CDRs comprising specific amino acid sequences in the light chain variable region (preferably the anti-RGMa neutralizing antibodies (a) to (1) described above).

The amino acid sequence of the anti-RGMa neutralizing antibody (preferably the anti-RGMa neutralizing antibodies (a) to (1) described above) may have substitution, deletion, addition or insertion of one or several (1 to 20, 1 to 10, or 1 to 5, but preferably 1 or 2) amino acids as long as the antibody of the present invention maintains the characteristics of having an ability to bind to RGMa and inhibiting (neutralizing) the activity of RGMa. The substitution, deletion, or addition may be introduced in CDRs, but it is preferably introduced in a region other than CDR. The amino acid substitution is preferably conservative substitution in order to maintain the characteristics of the present invention.

When the amino acid sequence of the anti-RGMa neutralizing antibody of the present invention (preferably the anti-RGMa neutralizing antibodies (a) to (1) described above) has a substitution, deletion, or the like therein, the amino acid sequence of the heavy chain variable region after the modification of the amino acid sequence has a % identity of 90% or more (more preferably 95%, 96%, 97%, 98%, or 99% or more) to the amino acid sequence before the modification, and the amino acid sequence of the light chain variable region after the modification of the amino acid sequence has a % identity of 90% or more (more preferably 95%, 96%, 97%, 98%, or 99% or more) to the amino acid sequence before the modification.

In the present invention the term "siRNA" refers to a short double strand RNA capable of inhibiting the expression of a target gene (a RGMa gene in the present invention). The nucleotide sequence and the length (base length) are not particularly limited as long as the function as an siRNA to inhibit the RGMa activity in the present invention is maintained. The base length is preferably less than about 30 bases, more preferably about 19 bases to 27 bases, and still more preferably about 21 bases to 25 bases.

In the present invention the term "shRNA" refers to a molecule with about 20 or more base pairs, which is a single strand RNA comprising a part having a palindromic nucleotide sequence to form a double-stranded structure in the molecule, and has a short hairpin structure with a 3'-terminal overhang. Such an shRNA can be introduced into a cell and then degraded into a length of about 20 bases (typically, for example, 21, 22, or 23 bases) in the cell to inhibit the expression of a target gene similarly to siRNA.

The siRNA and shRNA in the present invention may be in any form as long as they can inhibit the expression of the RGMa gene.

In the present invention, an siRNA or shRNA can be chemically synthesized by an artificial means. Antisense and sense RNAs can also be synthesized *in vitro* from DNA templates using, for example, a T7 RNA polymerase and a T7 promoter. The antisense oligonucleotide may be any nucleotide that is complementary to, or hybridizes to a consecutive nucleotide sequence having a length from 5 to 100 in the DNA sequence of a RGMa gene, and may be DNA or RNA. The antisense oligonucleotide may be modified insofar as its function is not impaired. The antisense oligonucleotide can be synthesized by a conventional method, and for example, it can be easily synthesized with a commercially available DNA synthesizer.

A preferred sequence can be selected by a common selection method, and the validation as the siRNA or shRNA according to the present invention can be made by evaluating inhibition of the expression of a functional RGMa.

<Diabetic Autonomic Neuropathy>.

Diabetic autonomic neuropathy as used herein means an autonomic nervous system disorder caused by chronic hyperglycemia such as diabetes, and an autonomic nervous system disorder that causes kidney dysfunction.

Here, examples of the autonomic nervous system disorder caused by chronic hyperglycemia such as diabetes include a wide variety of signs and symptoms seen in organs throughout the body caused by chronic hyperglycemia, such as:
(1) a cardiovascular autonomic neuropathy including orthostatic hypotension or arrhythmia; (2) a gastrointestinal autonomic neuropathy including vomiting or diarrhea (such as diabetic diarrhea) due to gastric paresis; (3) an autonomic neuropathy of urinary and genitourinary system including neurogenic bladder or erectile dysfunction; (4) an autonomic neuropathy of the metabolic system including asymptomatic hypoglycemia or hypoglycemia-related autonomic failure; and/or (5) an autonomic neuropathy related to peripheral vasomotor function including failure of blood pressure regulation mechanism caused by the disorder, disruption of fluid homeostasis due to endocrine system disorder, and/or anemia.

Since the kidney autonomic nervous system is deeply involved in the kidney function by regulating systemic and renal blood flow, secretion of neurohumoral factors, and direct action on the renal vasculature, protection of the kidney autonomic nervous system may contribute to the improvement of kidney function in the pathogenesis of chronic kidney disease in a wide range (Reference: Front Med. 2018 Mar 29;5:82. doi: 10.3389/fmed.2018.00082.). Accordingly, examples of autonomic neuropathy as a cause of kidney dysfunction include kidney diseases involved in kidney dysfunction, such as chronic kidney disease (for example, diabetic kidney disease (including diabetic nephropathy)).

The present invention is expected to have a preventive or therapeutic effect on the disadvantages (diseases or symptoms) brought about by the diabetic autonomic neuropathy described above.

A subject of treatment in the present invention (preferably a mammal, especially a human) is a patient who has developed diabetic autonomic neuropathy, and the diabetic autonomic neuropathy prevention or treatment agent of the present invention can be administered to such a patient.

In this regard, a "treatment" includes any treatment of a disease in a therapeutic objective, preferably a mammal, and especially a human, and includes prevention of progression of a disease or symptom so as to extinguish, heal, alleviate, or mitigate such a disease and symptom.

The term "prevention" also includes prevention or inhibition of the onset of the above diseases in a therapeutic objective, preferably a mammal, and especially a human. Furthermore, "prevention" in the present invention includes "recurrence prevention" which prevents recurrence of the above-mentioned diseases in which remission and recurrence are repeated in a therapeutic objective, preferably a mammal, and especially a human.

<Pharmaceutical composition>

The agent for preventing or treating diabetic autonomic neuropathy in the present invention is usually administered systemically or topically as oral or parenteral formulations.

The agent for preventing or treating diabetic autonomic neuropathy in the present invention can be made into a formulation by blending a pharmaceutically acceptable carrier or additive as appropriate using a RGMa inhibiting substance as an active ingredient. The thus formulated pharmaceutical composition can be administered in an oral or parenteral form. Specifically, the agent can be formed into oral agents such as tablets, coated tablets, pills, powders, granules, capsules, solutions, suspensions, and emulsions; or parenteral agents such as injections, infusions, suppositories, ointments, and patches. The content ratio of the carrier or additive may be set as appropriate according to the ranges commonly used in the pharmaceutical field. There is no particular restriction on the carrier or additive to be blended, and examples thereof include water, physiological saline, other aqueous solvents, various carriers such as aqueous or oily bases; and various additives such as excipients, binders, pH adjusters, disintegrants, absorption promoters, lubricants, colorants, flavoring agents, and perfumes.

When the RGMa inhibiting substance is an anti-RGMa neutralizing antibody, a functionally modified antibody thereof, a conjugated antibody thereof, or an antigen-binding fragment thereof, the agent is preferably formulated into an injection or infusion with a pharmaceutically acceptable carrier, and administered via a parenteral route of administration, such as an intravenous, intramuscular, intradermal, intraperitoneal, subcutaneous, or topical route.

The injection or infusion containing the anti-RGMa neutralizing antibody can be, for example, used as a solution, suspension, or emulsion. Examples of the solvent therefor include distilled water for injection, physiological saline, a glucose solution, and an isotonic solution (for example, solutions of sodium chloride, potassium chloride, glycerin, mannitol, sorbitol, boracic acid, borax, and propylene glycol).

The injections or infusions containing the anti-RGMa neutralizing antibody may further contain a stabilizer, a solubilizer, a suspending agent, an emulsifier, a soothing agent, a buffer agent, a preservative, an antiseptic, a pH adjuster, etc.

Examples of the stabilizer include albumin, globulin, gelatin, mannitol, glucose, dextran, ethylene glycol, propylene glycol, ascorbic acid, sodium bisulfite, sodium thiosulfate, sodium EDTA, sodium citrate, and dibutylhydroxytoluene.

Examples of the solubilizer include alcohols (such as ethanol), polyalcohols (such as propylene glycol and polyethylene glycol), and nonionic surfactants (such as polysorbate 80 (registered trademark), and HCO-50).

Examples of the suspending agent include glyceryl monostearate, aluminum monostearate, methylcellulose, carboxymethylcellulose, hydroxymethylcellulose, and sodium lauryl sulfate.

Examples of the emulsifier include gum arabic, sodium alginate, and tragacanth.

Examples of the soothing agent include benzylalcohol, chlorobutanol, and sorbitol.

Examples of the buffer agent include a phosphate buffer, acetate buffer, borate buffer, carbonate buffer, citrate buffer, and Tris buffer.

Examples of the preservative include methyl *para*-hydroxybenzoate, ethyl *para*-hydroxybenzoate, propyl *para*-hydroxybenzoate, butyl *para*-hydroxybenzoate, chlorobutanol, benzyl alcohol, benzalkonium chloride, sodium dehydroacetate, sodium edetate, boric acid, and borax.

Examples of the antiseptic include benzalkonium chloride, *para*-hydroxybenzoic acid, and chlorobutanol.

Examples of the pH adjuster include hydrochloric acid, sodium hydroxide, phosphoric acid, and acetic acid.

When the RGMa inhibiting substance is a nucleic acid (such as siRNA, shRNA, and antisense oligonucleotide), it can be administered in the form of a nonviral or viral vector. When it is in the form of a nonviral vector, a method of introducing a nucleic acid molecule using a liposome (such as a liposome method, an HVJ-liposome method, a cationic liposome method, a lipofection method, or a lipofectamine method), a microinjection method, a method of transferring a nucleic acid molecule with a carrier (a metal particle) into a cell using a gene gun or the like, can be used. When siRNA or shRNA is administered to a living organism using a viral vector, a viral vector such as a recombinant adenovirus or retrovirus can be used. A DNA that expresses the siRNA or shRNA is introduced to a DNA virus or RNA virus such as detoxified retrovirus, adenovirus, adeno-associated virus, herpesvirus, vaccinia virus, poxvirus, poliovirus, sindbis virus, Sendai virus, or SV40. By infecting a cell or a tissue with the recombinant virus, the gene can be introduced into the cell or tissue.

The formulation thus obtained can be administered to, for example, a human or another mammal (such as a rat, mouse, rabbit, sheep, pig, cattle, cat, dog, or monkey) at an effective dose to prevent or treat diabetic autonomic neuropathy. The dose is set as appropriate depending on the purpose, the severity of a disease, the age, weight, sex, and past history of a patient, the type of an active ingredient, or the like. For example, when the active ingredient is an anti-RGMa neutralizing antibody, the dose for an average human having a weight of about 65 kg to 70 kg is preferably about 0.02 mg to 4000 mg per day, more preferably about 0.1 mg to 200 mg per day. The total dose per day may be administered in a single dose or in divided doses.

<Combination with Other Drug or Treatment>

In the present invention, an agent for preventing or treating diabetic autonomic neuropathy may be administered in combination with an antidiabetic drug in order to control blood glucose level. A therapeutic agent for the antidiabetic drug to be used in combination includes, for example, blood glucose lowering agents, specificallyfor example, DPP4 inhibitors, SGLT inhibitors, GLP-1 receptor agonists.

An agent for preventing or treating diabetic autonomic neuropathy in the present invention may be administered in combination with a hypertension-treating agent in order to control blood pressure. Examples of the hypertension-treating agent to be used in combination include an angiotensin II receptor antagonist (ARB), and an ACE inhibitor and if antihypertensive effect is inadequate, a Ca antagonist or diuretic may be used in combination

The above other drug or treatment may be administered or performed before or after administration of an agent for preventing or treating diabetic autonomic neuropathy according to the present invention, or may be administered or performed at the same time.

EXAMPLES

The present invention will be described in more detail below with reference to Examples, which do not limit the scope of the present invention. As the anti-RGMa neutralizing antibody, an anti-RGMa neutralizing antibody comprising the amino acid sequences of (a) (SEQ ID NOS; 5 to 10) described herein was used in each example.

[Example 1]

Using a mouse model of drug-induced diabetes, the expression of RGMa mRNA in the kidney under diabetic conditions was analyzed, and then a therapeutic effect of anti-RGMa neutralizing antibody on kidney autonomic neuropathy was examined histologically.

< Induction of Diabetes >

Seven- to eight-week-old C57BL/6J female mice were used for experiments. The diabetes-induced group received a single intraperitoneal dose of 20 mg/ml streptozocin (STZ: Sigma-Aldrich) at a dose of 10 ml/kg. The non-diabetic induction group received a solvent at a dose of 10 ml/kg. Referring to previous studies (References 1, 2), blood glucose levels were measured one week after diabetes induction, and individuals with blood glucose levels less than 300 mg/dl were excluded.

< RGMa mRNA Expression Analysis >

At 8 weeks after induction of diabetes by STZ, mice (4 mice) and control mice (4 mice) were fully anesthetized, then abdominally opened, and ice-cold PBS was perfused through the left ventricle and de-bleeding was performed. The kidneys were removed as soon as possible, collected in tissue crush tubes (TM-625S; TOMY SEIKO Co., Ltd.) containing appropriate amounts of TRIzol solution (15596026; Thermo Fisher Scientific) and zirconia beads for crushing (ZB-10; TOMY SEIKO Co., Ltd.), and crushed using a bead cell disrupter (MS-100R; TOMY SEIKO Co., Ltd.). RNA was then extracted and purified (using RNeasy Mini Kit (74104; QIAGEN)), and cDNA was prepared by reverse transcription reaction, and a reaction was performed using Fast SYBR Green Master mix (4385612, Thermo Fisher Scientific) and QuantStudio 7 Flex Real-Time PCR System (Thermo Fisher Scientific). Relative quantification was performed using the $\Delta\Delta Ct$ method with Gapdh as an internal control, from the Ct values measured from the results.

< Histological Analysis: Antibody Administration Method, Grouping >

Purchased mice were randomly divided into 4 groups, "non-diabetic - anti-RGMa neutralizing antibody group", "non-diabetic - isotype control antibody (Palivizumab) group", "diabetic - anti-RGMa neutralizing antibody group", and "diabetic - isotype control antibody (Palivizumab) group", and administration of anti-RGMa neutralizing or control antibody was started 3 days after STZ (diabetic group) or solvent (citrate buffer (pH = 4.5)) administration. After adjusting the concentration of each antibody to 6 mg/ml, a dose of 30 mg/kg was administered intravenously into the tail vein once a week for a total of six times, and samples were collected after 6 weeks.

< Tissue Sampling, Renal Transparency, and Immunohistochemical Staining >

After adequate anesthesia, the kidneys were removed after perfusion fixation with 4% paraformaldehyde (PFA), and after postfixation, the kidneys were placed in a 30% sucrose/PBS solution at 4°C for 2 to 3 days and then clarified according to the CUBIC method. The following procedure modified after examination of conditions with reference to previous studies (References 3-7) was used. After 30% sucrose substitution, the kidneys were washed in PBS, transferred into CUBIC-L solution diluted to 50% with ultrapure water, and shaken overnight at room temperature. The kidneys were then transferred to 100% CUBIC-L solution and shaken at 37°C for 5 days. After washing with PBS, the kidneys were transferred to primary antibody solution containing 0.1% Triton X-100, 0.5% BSA, and 0.01% sodium azide in PBS, and shaken at 37°C for 5 days. After washing in PBS-0.5% Triton-X100 for 1 day, the kidneys were transferred into a secondary antibody solution diluted in PBS containing 0.1% Triton X-100, 0.1% BSA, and 0.01% sodium azide, and shaken at 37°C for 5 days. The kidneys were washed with PBS-0.5% Triton X-100 for 1 day, immersed in 1% formaldehyde solution diluted in PB (0.2 M) for 3 hours, and washed with PBS. Transparency was achieved by immersion in CUBIC-R diluted to 50% with ultrapure water for at least 6 hours and then in 100% CUBIC-R. A confocal laser microscope FV-3000 (Olympus) was used for observation and imaging of the transparency-enhanced tissue. A central area of the transparency-enhanced kidney, up to 200 μm from the surface, was imaged at a z-interval of 1 μm, and the captured images were projected onto a single image, and the density of TH-positive sympathetic nerve fibers per unit area was then quantified. This operation was performed on 6 individuals in each group, and analysis was performed. Reagents used were as follows.

CUBIC-L Solution

Triton X-100 (NACALAI TESQUE, INC., Kyoto, Japan): 10 w% N-buthyldiethanolamine (Tokyo Chemical Industry Co., Ltd, Tokyo, Japan): 10 w% The above-described reagents were dissolved in ultrapure water.

CUBIC-R solution

2,3-dimethyl-1-phenyl-5-pyrazolane/antipyrine (Tokyo Chemical Industry Co., Ltd, Tokyo, Japan): 45w% nicotinamide (Tokyo Chemical Industry Co., Ltd, Tokyo, Japan): 30 w%
The above-described reagents were dissolved in ultrapure water.

Primary antibody

Anti-tyrosine hydroxylase (TH) antibody (1:100; abcam, Cambridge, UK)

Secondary antibody

Alexa Fluor 488 donkey anti-sheep IgG (H+L) (1:200; Invitrogen, Waltham, MA, USA)

< Results >

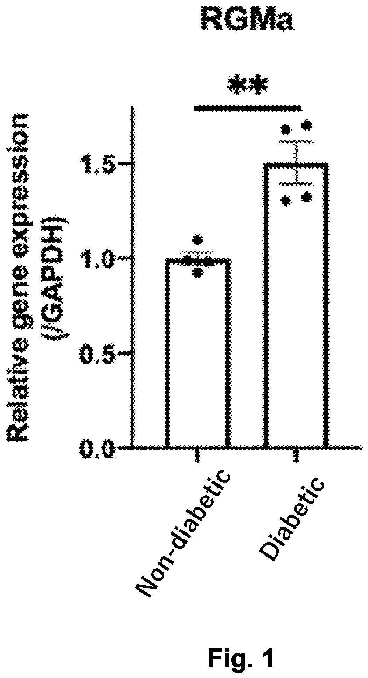

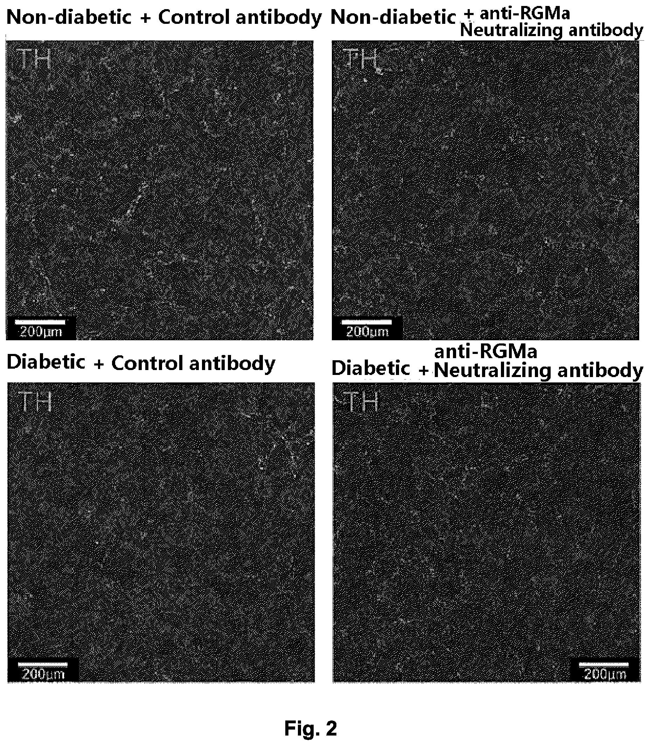

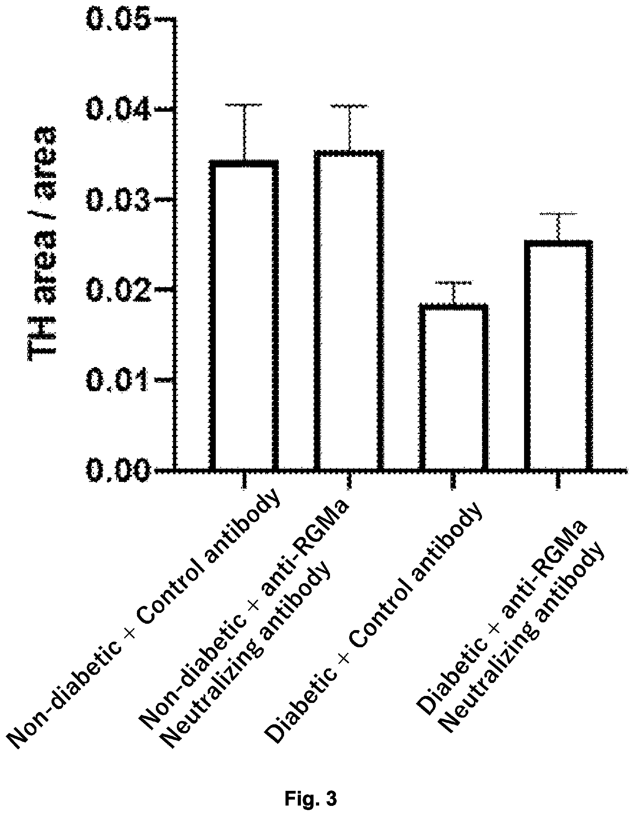

Gene expression analysis confirmed increased expression of RGMa mRNA in the kidney in diabetic pathology (Fig. 1), suggesting involvement of RGMa in this pathology. Representative staining images of anti-RGMa antibody and control administration experiments to diabetic mice conducted next (Fig. 2) and quantitative data of TH fiber density (Fig. 3) are shown. The results for the four groups shown in the quantitative data were obtained from n=6 for each group. For the non-diabetic group, no differences in TH fiber density were observed between the antibodies. On the other hand, TH fiber density tended to decrease in the "diabetes - isotype control antibody group" as compared to the non-diabetic group. On the other hand, the "diabetes - anti-RGMa neutralizing antibody group" exhibited a tendency to improve TH fiber density compared to the "diabetes - isotype control antibody group". These results suggest that a anti-RGM neutralizing antibody is considered to alleviate kidney autonomic neuropathy induced by diabetes.

[Example 2]

Using a mouse model of drug-induced diabetes, therapeutic effects of anti-RGMa neutralizing antibodies on kidney dysfunction were examined using urinary protein as an index.

< Induction of Diabetes >

Seven- to eight-week-old C57BL/6J female mice were used for experiments. The diabetes-induced group received a single intraperitoneal dose of streptozocin (STZ: Sigma-Aldrich) 20 mg/mL at a dose of 10 mL/kg. The non-diabetic induction group received a solvent at a dose of 10 mL/kg. Referring to previous studies (References 1, 2), blood glucose levels were measured three days after diabetes induction, and individuals with blood glucose levels less than 300 mg/dL were excluded.

< Histological Analysis: Antibody Administration Method, Grouping >

Purchased mice were randomly divided into 3 groups, "non-diabetic physiological saline group", "diabetes - anti-RGMa neutralizing antibody group", and "diabetes - isotype control antibody (Palivizumab) group", and administration of physiological saline, anti-RGMa neutralizing, or control antibody was started 3 days after STZ (diabetic group) or solvent (citrate buffer (pH = 4.5)) administration. Physiological saline was administered intravenously into the tail vein at a dose of 30 mg/kg once a week for a total of five times. After adjusting the concentration of each antibody to 6 mg/mL, a dose of 30 mg/kg was administered intravenously into the tail vein once a week for a total of five times, and samples were collected after 5 weeks.

< Calculation of Urinary Albumin/Urinary Creatinine Ratio >

The urinary albumin/urinary creatinine ratio was used as an indicator of proteinuria due to renal damage. At 5 weeks after diabetes induction, individuals of each group were placed in metabolic cages (Tecniplast Japan Co., Ltd., Tokyo, Japan), and urine samples were collected under ad libitum conditions. Samples were stored at -30°C until used for assay. Urinary albumin was quantified using LBIS® Albumin Mouse ELISA Kit (FUJIFILM Wako Shibayagi Corporation, Gunma pref., Japan) and urinary creatinine was quantified using Lab Assay™ Creatinine (FUJIFILM Wako Pure Chemical Corporation, Osaka, Japan), and the urinary albumin/urinary creatinine ratio (UACR) was calculated.

< Results >

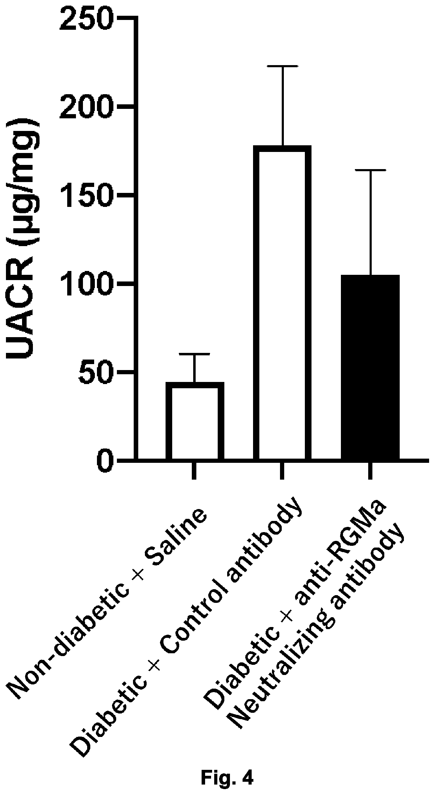

Quantitative data for urinary albumin/urinary creatinine ratio are shown in Fig. 4. Quantitative results for the three groups were obtained from n=6 for each group. The urinary albumin/urinary creatinine ratio tended to increase in the "diabetes - isotype control antibody group" as compared with the non-diabetic - saline group, which was considered to reflect renal damage. On the other hand, there was an improvement trend in the urinary albumin/urinary creatinine ratio in the "diabetes-anti-RGMa neutralizing antibody group" as compared to the "diabetes-isotype control antibody group". Therefore, an anti-RGMa neutralizing antibody is considered to have an alleviating effect on renal damage caused by diabetes. These results suggest that an anti-RGMa neutralizing antibody can alleviate kidney autonomic neuropathy induced by diabetes.

From the above, a RGMa inhibitor, preferably an anti-RGMa neutralizing antibody, can be expected as a preventive or therapeutic agent for autonomic neuropathy as a cause of kidney dysfunction, as well as for kidney disease involving kidney dysfunction, such as chronic kidney disease.

[References]

1. Deeds MC, Anderson JM, Armstrong AS, et al. Single dose streptozotocin-induced diabetes: Considerations for study design in islet transplantation models. LabAnim. 2011;45(3):131-140. doi:10.1258/la.2010.010090
2. O'brien PD, Sakowski SA, Feldman EL. Mouse models of diabetic neuropathy. ILAR J. 2014;54(3):259-272. doi:10.1093/ilar/ilt052
3. Hasegawa S, Susaki EA, Tanaka T, et al. Comprehensive three-dimensional analysis (CUBIC-kidney) visualizes abnormal renal sympathetic nerves after ischemia/reperfusion injury. Kidney Int. 2019;96(1): 129-138.doi:10.1016/j.kint.2019.02.011
4. Kubota SI, Takahashi K, Nishida J, et al. Whole-Body Profiling of Cancer Metastasis with Single-Cell Resolution. Cell Rep. 2017;20(1):236-250.doi:10.1016/j.celrep.2017.06.010
5. Tainaka K, Murakami TC, Susaki EA, et al. Chemical Landscape for Tissue Clearing Based on Hydrophilic Reagents. Cell Rep. 2018;24(8):2196-2210.e9.doi:10.1016/j.celrep.2018.07.056
6. Richardson DS, Lichtman JW. Clarifying Tissue Clearing. Cell. 2015;162(2):246-257. doi:10.1016/j.cell.2015.06.067
7. Yokoyama T, Lee JK, Miwa K, et al. Quantification of sympathetic hyperinnervation and denervation after myocardial infarction by three-dimensional assessmentof the cardiac sympathetic network in cleared transparent murine hearts. PLoS One. 2017;12(7):1-13. doi:10.1371/journal.pone.0182072

<Sequence Listing>

SEQ ID NO: 1: Amino acid sequence of human RGMa precursor protein

SEQ ID NO: 2: Amino acid sequence of mouse RGMa precursor protein

SEQ ID NO: 3: Amino acid sequence of rat RGMa precursor protein

SEQ ID NO: 4: DNA sequence of human RGMa gene

SEQ ID NO: 5: Amino acid sequence of LCDR1 of anti-RGMa neutralizing antibody r116A3

SEQ ID NO: 6: Amino acid sequence of LCDR2 of anti-RGMa neutralizing antibody r116A3

SEQ ID NO: 7: Amino acid sequence of LCDR3 of anti-RGMa neutralizing antibody r116A3

SEQ ID NO: 8: Amino acid sequence of HCDR1 of anti-RGMa neutralizing antibody r116A3

SEQ ID NO: 9: Amino acid sequence of HCDR2 of anti-RGMa neutralizing antibody r116A3

SEQ ID NO: 10: Amino acid sequence of HCDR3 of anti-RGMa neutralizing antibody r116A3

SEQ ID NO: 11: Amino acid sequence of LCDR1 of anti-RGMa neutralizing antibody r70E

SEQ ID NO: 12: Amino acid sequence of LCDR2 of anti-RGMa neutralizing antibody r70E

SEQ ID NO: 13: Amino acid sequence of LCDR3 of anti-RGMa neutralizing antibody r70E

SEQ ID NO: 14: Amino acid sequence of HCDR1 of anti-RGMa neutralizing antibody r70E

SEQ ID NO: 15: Amino acid sequence of HCDR2 of anti-RGMa neutralizing antibody r70E

SEQ ID NO: 16: Amino acid sequence of human RGMa epitope

SEQ ID NO: 17: Amino acid sequence of LCDR1 of anti-RGMa neutralizing antibody 5F9

SEQ ID NO: 18: Amino acid sequence of LCDR2 of anti-RGMa neutralizing antibody 5F9

SEQ ID NO: 19: Amino acid sequence of LCDR3 of anti-RGMa neutralizing antibody 5F9

SEQ ID NO: 20: Amino acid sequence of HCDR1 of anti-RGMa neutralizing antibody 5F9

SEQ ID NO: 21: Amino acid sequence of HCDR2 of anti-RGMa neutralizing antibody 5F9

SEQ ID NO: 22: Amino acid sequence of HCDR3 of anti-RGMa neutralizing antibody 5F9

SEQ ID NO: 23: Amino acid sequence of LCDR1 of anti-RGMa neutralizing antibody 8D1

SEQ ID NO: 24: Amino acid sequence of LCDR2 of anti-RGMa neutralizing antibody 8D1

SEQ ID NO: 25: Amino acid sequence of LCDR3 of anti-RGMa neutralizing antibody 8D1

SEQ ID NO: 26: Amino acid sequence of HCDR1 of anti-RGMa neutralizing antibody 8D1

SEQ ID NO: 27: Amino acid sequence of HCDR2 of anti-RGMa neutralizing antibody 8D1

SEQ ID NO: 28: Amino acid sequence of HCDR3 of anti-RGMa neutralizing antibody 8D1

SEQ ID NO: 29: Amino acid sequence of LCDR1 of anti-RGMa neutralizing antibody AE12-1

SEQ ID NO: 30: Amino acid sequence of LCDR2 of anti-RGMa neutralizing antibody AE12-1

SEQ ID NO: 31: Amino acid sequence of LCDR3 of anti-RGMa neutralizing antibody AE12-1

SEQ ID NO: 32: Amino acid sequence of HCDR1 of anti-RGMa neutralizing antibody AE12-1

SEQ ID NO: 33: Amino acid sequence of HCDR2 of anti-RGMa neutralizing antibody AE12-1

SEQ ID NO: 34: Amino acid sequence of HCDR3 of anti-RGMa neutralizing antibody AE12-1

SEQ ID NO: 35: Amino acid sequence of LCDR3 of anti-RGMa neutralizing antibody AE12-1Y

SEQ ID NO: 36: Amino acid sequence of human RGMa epitope

SEQ ID NO: 37: Amino acid sequence of human RGMa epitope

SEQ ID NO: 38: Amino acid sequence of human RGMa epitope

SEQ ID NO: 39: Amino acid sequence of human RGMa epitope

SEQ ID NO: 40: Amino acid sequence of LCDR3 of anti-RGMa neutralizing antibody AE12-1F

SEQ ID NO: 41: Amino acid sequence of LCDR3 of anti-RGMa neutralizing antibody AE12-1H

SEQ ID NO: 42: Amino acid sequence of LCDR3 of anti-RGMa neutralizing antibody AE12-1L

SEQ ID NO: 43: Amino acid sequence of LCDR3 of anti-RGMa neutralizing antibody AE12-1V

SEQ ID NO: 44: Amino acid sequence of LCDR3 of anti-RGMa neutralizing antibody AE12-1I

SEQ ID NO: 45: Amino acid sequence of LCDR3 of anti-RGMa neutralizing antibody AE12-1K

## INDUSTRIAL APPLICABILITY

Since the RGMa inhibiting substance is useful for preventing or treating diabetic autonomic neuropathy, the present invention has high utility in the pharmaceutical industry.

## Claims

1. An agent for preventing or treating diabetic autonomic neuropathy comprising a RGMa inhibiting substance.

2. The preventive or therapeutic agent according to claim 1, wherein the diabetic autonomic neuropathy is autonomic neuropathy caused by chronic hyperglycemia or autonomic neuropathy which causes kidney dysfunction.

3. The preventive or therapeutic agent according to claim 1 or 2, wherein the diabetic autonomic neuropathy is autonomic neuropathy which causes kidney dysfunction.

4. The preventive or therapeutic agent according to any one of claims 1 to 3, wherein the diabetic autonomic neuropathy is a kidney disease which is involved in kidney dysfunction.

5. The preventive or therapeutic agent according to claim 4, wherein the kidney disease which is involved in kidney dysfunction is a chronic kidney disease.

6. The preventive or therapeutic agent according to any one of claims 1 to 5, wherein the RGMa inhibiting substance is an anti-RGMa neutralizing antibody.

7. The preventive or therapeutic agent according to claim 6, wherein the anti-RGMa neutralizing antibody is a humanized antibody.

8. The preventive or therapeutic agent according to claim 6 or 7, wherein the anti-RGMa neutralizing antibody is an antibody recognizing an amino acid sequence selected from SEQ ID NOS: 16, 36, 37, 38 and 39.

9. The preventive or therapeutic agent according to any one of claims 6 to 8, wherein the anti-RGMa neutralizing antibody is an antibody selected from the following (a) to (1):

(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9, and an HCDR3 comprising the amino acid sequence represented by SEQ ID NO: 10;
(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15, and an HCDR3 comprising an amino acid sequence comprising SFG;
(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;

(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27, and an HCDR3 comprising the amino acid sequence represented by SEQ ID NO: 28;

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and

(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34.

10. A method of preventing or treating diabetic autonomic neuropathy, which comprises administration of an effective dose of a RGMa inhibiting substance to a mammal in need of treatment.

11. The preventive or therapeutic method according to claim 10, wherein the RGMa inhibiting substance is an anti-RGMa neutralizing antibody.

12. Use of a RGMa inhibiting substance in manufacture of an agent for preventing or treating diabetic autonomic neuropathy.

13. The use according to claim 12, wherein the RGMa inhibiting substance is an anti-RGMa neutralizing antibody.

Fig. 1

**Non-diabetic + Control antibody**  **Non-diabetic + anti-RGMa Neutralizing antibody**

**Diabetic + Control antibody**  **Diabetic + anti-RGMa Neutralizing antibody**

**Fig. 2**

**Fig. 3**

**Fig. 4**

**INTERNATIONAL SEARCH REPORT**

International application No.

PCT/JP2021/001261

A. CLASSIFICATION OF SUBJECT MATTER
A61K 45/00(2006.01)i; A61K 39/395(2006.01)i; A61P 13/12(2006.01)i; A61P
25/02(2006.01)i; A61P 43/00(2006.01)i; C12N 15/13(2006.01)i
FI:     A61K45/00; A61K39/395 N; A61P43/00 111; A61P25/02 103; A61P13/12;
        C12N15/13 ZNA
According to International Patent Classification (IPC) or to both national classification and IPC

B. FIELDS SEARCHED

Minimum documentation searched (classification system followed by classification symbols)
A61K45/00; A61K39/395; A61P13/12; A61P25/02; A61P43/00; C12N15/13

Documentation searched other than minimum documentation to the extent that such documents are included in the fields searched
Published examined utility model applications of Japan          1922–1996
Published unexamined utility model applications of Japan        1971–2021
Registered utility model specifications of Japan                1996–2021
Published registered utility model applications of Japan        1994–2021

Electronic data base consulted during the international search (name of data base and, where practicable, search terms used)
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2016/175236 A1 (MITSUBISHI TANABE PHARMA CORPORATION) 03 November 2016 (2016-11-03) claims 1, 13, 14, 17, examples 15, 16 | 1–13 |
| Y | KORECKA, Joanna A. et al., "Repulsive Guidance Molecule a (RGMa) Induces Neuropathological and Behavioral Changes That Closely Resemble Parkinson's Disease", J Neurosci., 27 September 2017, vol. 37, no. 39, pp. 9361–9379 section "RGMa decreases neuronal density in the SN", fig. 6 | 1–13 |
| Y | JUNGEN, Christiane et al., "Increased arrhythmia susceptibility in type 2 diabetic mice related to dysregulation of ventricular sympathetic innervation", Am J Physiol Heart Circ Physiol, 18 October 2019, vol. 317, p. H1328–H1341 abstract, fig. 6 | 1–13 |

☐ Further documents are listed in the continuation of Box C.          ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

Date of the actual completion of the international search
12 March 2021 (12.03.2021)

Date of mailing of the international search report
23 March 2021 (23.03.2021)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**
Information on patent family members

International application No.

PCT/JP2021/001261

| Patent Documents referred in the Report | Publication Date | Patent Family | Publication Date |
|---|---|---|---|
| WO 2016/175236 A1 | 03 Nov. 2016 | US 2018/0100012 A1 claims 1, 13, 14, 17, examples 15, 16 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005087268 A
- WO 2009106356 A
- WO 2013112922 A
- WO 2016175236 A
- WO 2005035586 A
- WO 200231140 A
- WO 0061739 A
- US 6737056 B
- US 7297775 B
- US 7317091 B
- US 4737456 A
- JP 4506458 W
- JP 2912618 B
- JP 62296890 A
- JP 4504365 W
- JP 7509137 W
- WO 9425585 A
- JP 6500233 W

### Non-patent literature cited in the description

- *Diabetes Care,* 2003, vol. 26, 1553-1579
- *Journal of the Japanese Society for Dialysis Therapy,* 1986, vol. 19 (9), 905-909
- *Journal of the Japan Diabetes Society,* 1984, vol. 27 (6), 715-721
- *Am J Kidney Dis,* 2007, vol. 49, 12-154
- *vidence-based CKD Medical Guideline,* 2018, 104-105
- *Neuron,* 1990, vol. 5, 735-743
- *Philos. Trans. R. Soc. Lond. B Biol. Sci.,* 2006, vol. 361, 1513-29
- *Biochem. Biophys. Res. Commun.,* 2009, vol. 382, 795-800
- *Curr. Opin. Neurobiol.,* 2007, vol. 17, 29-34
- *J. Cell Biol.,* 2006, vol. 173, 47-58
- **KABAT et al.** Sequences of Proteins of Immunological Interest. US Department of Health and Human Services, 1987
- Current Protocols in Molecular Biology. John Wiley & Sons, 1987
- Antibodies: A Laboratory Manual. Cold Spring Harbor Laboratory, 1988
- **KÖHLER ; MILSTEIN et al.** *Nature,* 1975, vol. 256, 495
- *Nucleic Acids Research,* 1986, vol. 14, 1779
- *The Journal of Biological Chemistry,* 1982, vol. 257, 1516
- *Cell,* 1980, vol. 22, 197
- **HASHIGUCHI SHUHEI et al.** *Seikagaku,* 2010, vol. 82 (8), 710
- **D. J. KING.** Applications and Engineering of Monoclonal Antibodies. T.J. International Ltd, 1998
- Monoclonal Antibody-Based Therapy of Cancer. Marcel Dekker Inc, 1998
- **CHARI et al.** *Cancer Res.,* 1992, vol. 152, 127
- **LIU et al.** *Proc Natl Acad Sci USA.,* 1996, vol. 93, 8681
- *Nature Genetics,* 1994, vol. 7, 13-21
- *Nature Genetics,* 1997, vol. 15, 146-156
- *Nature,* 1994, vol. 368, 856-859
- **DEEDS MC ; ANDERSON JM ; ARMSTRONG AS et al.** Single dose streptozotocin-induced diabetes: Considerations for study design in islet transplantation models. *LabAnim,* 2011, vol. 45 (3), 131-140
- **O'BRIEN PD ; SAKOWSKI SA ; FELDMAN EL.** Mouse models of diabetic neuropathy. *ILAR J,* 2014, vol. 54 (3), 259-272
- **HASEGAWA S ; SUSAKI EA ; TANAKA T et al.** Comprehensive three-dimensional analysis (CUBIC-kidney) visualizes abnormal renal sympathetic nerves after ischemia/reperfusion injury. *Kidney Int.,* 2019, vol. 96 (1), 129-138
- **KUBOTA SI ; TAKAHASHI K ; NISHIDA J et al.** Whole-Body Profiling of Cancer Metastasis with Single-Cell Resolution. *Cell Rep,* 2017, vol. 20 (1), 236-250
- **TAINAKA K ; MURAKAMI TC ; SUSAKI EA et al.** Chemical Landscape for Tissue Clearing Based on Hydrophilic Reagents. *Cell Rep,* 2018, vol. 24 (8), 2196-2210.e9
- **RICHARDSON DS ; LICHTMAN JW.** Clarifying Tissue Clearing. *Cell,* 2015, vol. 162 (2), 246-257
- **YOKOYAMA T ; LEE JK ; MIWA et al.** Quantification of sympathetic hyperinnervation and denervation after myocardial infarction by three-dimensional assessment of the cardiac sympathetic network in cleared transparent murine hearts. *PLoS One,* 2017, vol. 12 (7), 1-13